Europäisches Patentamt

European Patent Office

Office européen des brevets

(19)

(11) Veröffentlichungsnummer: **0 054 814**
**B1**

(12) # EUROPÄISCHE PATENTSCHRIFT

(45) Veröffentlichungstag der Patentschrift:
21.08.85

(51) Int. Cl.⁴: **C 12 M 1/08**

(21) Anmeldenummer: 81110195.5

(22) Anmeldetag: 05.12.81

(54) **Verfahren zum Verbessern der Gasverteilung in Mammut-Schlaufen-reaktoren.**

(30) Priorität: **13.12.80 DE 3047101**

(43) Veröffentlichungstag der Anmeldung:
**30.06.82 Patentblatt 82/26**

(45) Bekanntmachung des Hinweises auf die Patenterteilung:
**21.08.85 Patentblatt 85/34**

(84) Benannte Vertragsstaaten:
**AT CH DE FR GB IT LI NL SE**

(56) Entgegenhaltungen:
**BE - A - 834 949**
**FR - A - 2 156 376**
**FR - A - 2 404 045**

(73) Patentinhaber: **HOECHST AKTIENGESELLSCHAFT,**
**Postfach 80 03 20, D-6230 Frankfurt am Main 80 (DE)**

(72) Erfinder: **Präve, Paul, Dr., Pfarrstrasse 5, D-6232 Bad**
**Soden am Taunus (DE)**
Erfinder: **Sittig, Wolfgang, Sulzbacher Weg 2,**
**D-6238 Hofheim am Taunus (DE)**

BUNDESDRUCKEREI BERLIN

**0 054 814**

**Beschreibung**

Gegenstand der Erfindung ist ein Verfahren zum Verbessern der Gasverteilung in Mammut (air lift)-Schlaufenreaktoren.

Es sind Gas-Flüssigkeitsreaktionen bekannt, bei denen die Flüssigkeiten in einer Schlaufe geführt werden. Es ist ferner bekannt, die Flüssigkeiten mit Gas anzutreiben. Die dafür verwendeten Reaktoren sind als air lift- bzw. Mammut-Schlaufenreaktoren bekannt. Das die Flüssigkeit durchströmende und antreibende Gas wird zum Beispiel mittels einfachen Düsen, Ringdüsen, Düsenringen, Begasungsrührern, Treibstrahldüsen, Ejektor-, Injektor-, Kegeldüsen und anderen mehr in die Flüssigkeit verteilt. Es ist weiterhin bekannt, daß die Blasenerzeugung und die Wirksamkeit der Blasenverteilung durch die Verwendung von mehreren Austrittsorganen und/oder durch zusätzliche Fremdenergiezufuhr verbessert werden kann.

Bei der Anordnung mehrerer Gasaustrittsorgane verteilt über den Reaktorquerschnitt besteht die Gefahr, daß die hintereinander austretenden Blasen eine Kette bilden, daß nachfolgende Blasen im Sog der voransteigenden beschleunigt werden, diese erreichen und mit ihnen verschmelzen, so daß die verfügbare Stoffaustauschfläche vermindert wird. Eine zusätzliche Zufuhr von Energie, zum Beispiel in Form von Flüssigkeiten, an den Gasaustrittsstellen bewirkt eine Verringerung der Blasengröße durch früheres Abreißen der Blase von der Austrittsöffnung und eine schnelle Vermischung der Blasen mit einem größeren Flüssigkeitsvolumen. Dadurch werden die Wahrscheinlichkeit eines Blasenkontaktes und damit die Koalenzrate herabgesetzt. Zusätzliche Energie mindert jedoch die Wirtschaftlichkeit solcher Verfahren.

Die Aufgabe der vorliegenden Erfindung besteht demnach darin, an alle Gaseinleitstellen einen energiereichen Flüssigkeitsstrom heranzuführen, ohne den Einsatz von zusätzlicher Energie zum Antreiben der Flüssigkeit.

Die Aufgabe wird durch ein Verfahren gelöst, das dadurch gekennzeichnet ist, daß die rückströmende, teilentgaste Flüssigkeit vor, bei oder nach Eintritt in den Aufstiegsteil der Schlaufe in Teilströme aufgeteilt wird, wobei der Querschnitt der Summe der Teilströme kleiner ist als der freie Querschnitt des Aufstiegsteils, und daß das Gas in die Teilströme eingeleitet wird. Dabei kann die Summe der Querschnitte der einzelnen Teilströme vorteilhaft das 0,05- bis 0,8fache des freien Querschnitts des Aufstiegsteils der Schlaufe betragen. Das entspricht Flüssigkeitsgeschwindigkeiten am Gaseintritt von 4 bis 100 m $\cdot$ sec$^{-1}$. Sind für die Reaktion weitere Komponenten, zum Beispiel Katalysatoren, erforderlich, ist es zweckmäßig, dieselben ebenfalls an den Gaseinleitstellen der Flüssigkeit zuzuführen. In besonderen Fällen kann es zweckmäßig sein, die erreichten Geschwindigkeiten der Teilströme durch einen Flüssigkeitstreibstrahl oder durch mechanische Mittel weiterzuerhöhen. Es kann ferner zweckmäßig sein, nur einen Teil der im Abstromteil abwärts strömenden, teilentgasten Flüssigkeit in Teilströme aufzuteilen und den Gaseinleitstellen zuzuführen.

Die Erfindung wird nun anhand der schematischen Darstellung näher erläutert:

In einen Mammut-Schlaufenreaktor, dessen zylindrischer oder ringspaltförmiger oder rechteckiger, begaster Aufstiegsraum (1) durch eine Leiteinrichtung (8) vom ringspaltförmigen oder zylindrischen oder rechteckigen, unbegasten oder schwach begasten Abstromraum (2) abgetrennt ist und dessen oberer Teil als Gasabscheidezone (7) ausgebildet ist, wird die Flüssigkeit durch die Leitung (9) zu und durch die Leitung (6) abgeführt. Die Begasung findet vorzugsweise am unteren Ende des Aufstiegsraumes (1) statt. Die teilentgaste nach unten strömende Flüssigkeit im Abstromraum (2) wird vor oder bei Eintritt in den unteren Teil des Aufstiegsraums (1) durch Einbauten (3) aufgestaut und in so viele Teilströme (4) großer Geschwindigkeit aufgeteilt, wie die Anzahl der Gaseinleitstellen (5) entspricht. Jeder Teilstrom (4) wird an je einer Gaseinleitstelle (5) in den Aufstiegsraum (1) eingeführt, mit dem Gasstrom gemischt, wodurch letzterer in den Aufstiegsraum verteilt wird. Es ist zweckmäßig, die Gaseinleitstelle nach Art einer Düse auszubilden und den Teilgasstrom und das Gas dort in die Flüssigkeit einzuführen, wo die Teilströme ihre höchste Geschwindigkeit aufweisen.

<p style="text-align:center">Beispiel 1</p>

Ein Schlaufenreaktor zylindrischen Querschnitts von 600 mm Durchmesser und 1,70 m Flüssigkeitshöhe, mit einem konzentrischen Innenrohr von 400 mm Durchmesser als Aufstiegsraum und dem Ringspalt als Abstromraum wurde mit 192 m³/h Luft begast. Bei Einsatz von drei Begasungsdüsen ließ sich ein Sauerstoffübergangskoeffizient von 210 h$^{-1}$ erzielen, bei Einsatz einer erfindungsgemäßen Vorrichtung betrug der Übergangskoeffizient 260 h$^{-1}$.

<p style="text-align:center">Beispiel 2</p>

Ein Schlaufenreaktor von 600 mm Durchmesser, 10 m Flüssigkeitshöhe, 500 mm Leitrohrdurchmesser zur Fermentation von Trichoderma viride wurde mit 300 m³/h Luft begast und erzielte einen Sauerstoffeintrag von 11 kg/h mit Begasung über eine Ringdüse. Bei Begasung mit drei Luftdüsen, die erfindungsgemäß in 3 Flüssigkeitsdüsen von 150 mm engstem Querschnitt angeordnet waren, erzielte die gleiche Luftmenge von 300 m$^{-3}$/h einen Sauerstoffeintrag von 15 kg/h.

Beispiel 3

Ein Schlaufenreaktor von 1600 mm Durchmesser, 17 m begaster Flüssigkeitshöhe und 1300 mm Innenrohrdurchmesser wurde zur Fermentation von Methylomonas clara eingesetzt. Die Nährlösung enthielt:

| | | |
|---|---|---|
| $H_3PO_4$ 75% | (techn. rein) | 0.2% |
| $K_2SO_4$ | (techn. rein) | 0.117% |
| $MgSO_4 \cdot 7H_2O$ | (techn. rein) | 0.08% |
| $FeSO_4 \cdot 7H_2O$ | (techn. rein) | 0.003% |
| $Na_2SO_4$ | (techn. rein) | 0.024% |
| Spurenelemente | (techn. rein) | 0.0001% (pH 2—3) |
| 25% $NH_3$-Wasser-pH-controlled | | 0.3% |
| Methanol | | 0.1% |

Die Nährlösung wurde mit einer Verdünnungsrate von 0.23 h$^{-1}$ zudosiert und die überlaufende Kulturmenge kontinuierlich abgeerntet. Bei Einsatz einer herkömmlichen Ringdüse von 80 mm Durchmesser mit einem Spalt von 8 mm wurde ein Sauerstoffeintrag von 120 kg/h erzielt, die eindosierte Frischluftmenge betrug 1700 m$^3$/h. Es wurde eine Produktion von 69 kg/h Biomasse erzielt. Durch erfindungsgemäße Aufteilung des entgasten Flüssigkeitsstromes in 7 Teilströme und einer Querschnittsverengung auf 0.04 m$^2$ je Teilstrom und Aufteilung der Gasmenge auf 7 Teilströme von je 240 m$^3$/h ergab sich ein Sauerstoffeintrag von 160 kg/h und eine Produktion von 92 kg/h. Die Fermentationstemperatur betrug 39° C, der pH 6,8.

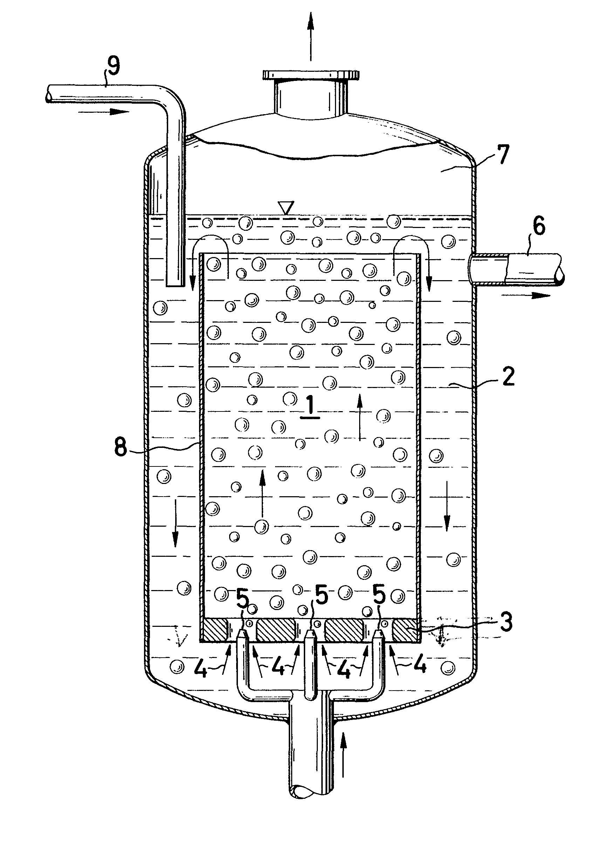

## Patentansprüche

1. Verfahren zum Verbessern der Gasverteilung in Mammut-Schlaufenreaktoren, dadurch gekennzeichnet, daß die rückströmende, teilentgaste Flüssigkeit vor, bei oder nach Eintritt in den Aufstiegsteil der Schlaufe in Teilströme aufgeteilt wird, wobei der Querschnitt der Summe der Teilströme kleiner ist als der freie Querschnitt des Aufstiegsteils, und daß das Gas in die Teilströme eingeleitet wird.

2. Verfahren nach Anspruch 1, dadurch gekennzeichnet, daß die Flüssigkeit in Teilströme aufgeteilt wird, deren Querschnittsumme, das 0,05 bis 0,8fache des freien Querschnitts des Aufstiegsteils der Flüssigkeit beträgt.

3. Verfahren nach den Ansprüchen 1 bis 2, dadurch gekennzeichnet, daß an den Gaseinleitstellen den Teilströmen weitere Komponenten zugeführt werden.

4. Verfahren nach Ansprüchen 1 bis 3, dadurch gekennzeichnet, daß die durch die Mammut-Pumpenwirkung erreichbaren Geschwindigkeiten der Teilströme durch einen Flüssigkeitstreibstrahl oder durch mechanische Mittel erhöht werden.

## Claims

1. A process for improving the gas distribution in air-lift loop reactors, which comprises dividing the back-flowing, partially degassed liquid into part streams prior to, during, or after entry into the rising part of the loop, the cross-section of the sum of the part streams being smaller than the free cross-section of the rising part, and passing to gas into the part streams.

2. A process as claimed in claim 1, wherein the liquid is divided into part streams, the sum of the cross-sections of which is 0.05- to 0.8 times the free cross-section of the rising part of the liquid.

3. A process as claimed in claims 1 to 2, wherein further components are passed into the part streams at the gas inlet points.

4. A process as claimed in any of claims 1 to 3, wherein the velocities of the part streams which can be achieved by the effect of the air-lift pump are increased by means of a liquid jet or by mechanical means.

## Revendications

1. Procédé pour améliorer la réparation de gaz dans des réacteurs mammouth à boucles, caractérisé en ce que le liquide partiellement dégazé qui s'écoule en retour est divisé, avant, dans ou après l'entrée dans la partie ascendante de la boucle en des courants partiels, la section de la somme des courants partiels étant plus petite que la section libre de la partie ascendante, et en ce que le gaz est introduit dans les courants partiels.

2. Procédé selon la revendication 1, caractérisé en ce que le liquide est divisé en courants partiels dont la somme des sections est comprise entre 0,05 et 0,8 fois la section libre de la partie ascendante du liquide.

3. Procédé selon une des revendications 1 et 2, caractérisé en ce que d'autres composants sont introduits dans les courants partiels dans les zones d'entrée de gaz.

4. Procédé selon les revendications 1 à 3, caractérisé en ce que les vitesses des courants partiels pouvant être obtenues par l'action de pompage mammouth sont augmentées par un jet propulseur de liquide ou par des moyens mécaniques.

0 054 814

1/1

5